# EUROPEAN PATENT SPECIFICATION

(11) **EP 0 280 798 B1**
(45) Date of publication and mention of the grant of the patent: **27.01.1993**
(21) Application number: 87301785.9
(22) Date of filing: 02.03.1987
(51) Int. Cl.: A61B 17/39

(54) **Electrosurgery surgical instrument**
Elektrochirurgisches Instrument
Instrument électrochirurgical

(43) Date of publication of application: 07.09.1988
(73) Proprietor: Everest Medical Corporation, Brooklyn Center Minnesota 55430 (US)
(72) Inventor: Stasz, Peter, Minneapolis Minnesota 55432 (US)
(74) Representative: MacGregor, Gordon

(56) References cited:
- WO-A-85/00280
- FR-A- 2 303 515
- FR-A- 2 303 517
- GB-A- 2 037 167
- US-A- 2 310 844
- US-A- 3 884 237
- US-A- 4 640 279

## Description

The present invention relates to a surgical instrument.

Various prior art electro-surgery and electro-cautery blades have been less effective in that either a special material is required for the blades, or they comprise a combination of structures which are primarily ornamental and non-functional, and which require a considerable drive voltage which often cause undue tissue damage.

Some of the prior art devices do not allow for appropriate coupling of their blade member to their voltage sources and tend to be ineffectual.

Other types of prior art blades utilise extremely elaborate and complex electrical circuits with standard type of blade structures which do not, in the end, accomplish a desirable result of an effective surgical blade capable of operation in more than one mode.

One of the most severe problems with the prior art is the adhesion of charred tissue and blood to the blade which shorts out the two conductors, and therefore, renders the blade useless as an electro-surgical device.

The invention disclosed in International Publication Nº WO 85/00280 concerns a tool for performing electro-cautery and/or electro-surgical procedures which, by utilising state-of-the-art semiconductor masking technology, can be designed to function as a standard surgical blade, or as an electrified blade.

The present invention provides an electro-surgical instrument comprising an electro-surgical blade means for electrically cutting and cauterising tissue, characterised by means for vibrating the surgical blade means at a frequency and amplitude to prevent the build-up of tissue debris thereon.

In one embodiment of the present invention there is also provided a surgical tool which includes a capacitive blade. In an alternative embodiment of the present invention, there is provided a piezo-ceramic, or other like element, coupled to a surgical blade for vibrating the blade so as to provide a cavitation effect which cleans the blade continuously during use.

According to a preferred embodiment of the invention there is provided an electro-cautery/electro-surgical tool which can be utilised as a standard surgical cutting blade with a sharp edge when no electrical power is applied to it; as an electro-surgical blade when a high voltage is applied between conductive surfaces sufficient to create a discharge for cutting and cauterizing of tissue; and as a low voltage electro-cautery tool where I²R losses heat the blade to cauterize the tissue.

According to another preferred embodiment of the present invention there is provided an electro-surgical tool utilising a capacitive type blade in which two conductors are separated by an insulator, and driven by an alternating current.

According to the present invention, and irrespective of the particular blade style, the present invention incorporates means like a piezo-ceramic or other transducer element for vibrating the blade sufficient to produce a cavitation effect, thereby preventing build up of tissue debris on the blade. The transducer may be affixed to the blade member or disposed in the handle member in intimate contact with the blade so as to vibrate the blade producing cavitation at the interface between the tissue and the blade.

In one embodiment of the present invention, there is provided an electro-cautery surgical tool, including a base member of a conductive material, such as stainless steel or the like, the base material fashioned in the form of a surgical blade with a sharpened edge leading to a point, layers of insulation disposed on opposing sides of the blade, a second layer of conductive material deposited over the insulation material, and a plurality of geometrical gaps extending through the second conductive layer which may be formed by semiconductor technology techniques including sandblasting by masking, laser machining, chemical action, electrode discharge machining (EDM), electron beam drilling, ion milling, or grinding, to define a conductive comb-shaped electrodes on the opposing sides of the blade. Alternative embodiments of the present invention include opposing gaps on opposing surfaces of the blade, staggered gaps on opposing surfaces of the blade, holes partially through the second conductive portion to the first conductive portion of the blade, and a sandwiched alternating configuration of gaps.

It is also possible to have an electro-surgical tool which includes a conductive blade, a thick insulation layer extending across the conductive blade except at a defined sharpened cutting edge of the blade, a narrow conductor, such as a foil or the like, positioned slightly offset from the edge of the thick insulation, and a final thinner layer insulation covering the narrow conductor whereby the narrow conductor and the underlying blade conductor provide a capacitive effect for bioelectrically breaking down flesh.

An electrical-to-mechanical transducer, such as a piezo-ceramic, such as barium titanate, can be attached to or mechanically coupled to the surgical blade for vibrating the blade so as to provide a cavitation effect.

One preferred embodiment of the present invention is an electro-cautery/electro-surgical blade which can be utilised in a plurality of modes including a standard surgical blade, an electro-surgical blade mode, or a mode where the blade is heated during cauterization.

It is possible to have an electro-cautery/electro-surgical blade which uses state-of-the-art technology in producing the blade at minimized cost, thereby making the blade disposable.

Further, it is possible to have an electro-cautery/electro-surgical blade which utilises a capacitive relationship between two conductors.

It is an object hereof to provide an improved electro-cautery/electro-surgical blade.

The principal object is to provide an electro-cautery/electro-surgical blade which is ultrasonically vibrated.

Further, it is possible to have a surgical blade which is disposable and produced by state-of-the-art semiconductor integrated circuit manufacturing processes.

Yet it is also possible to have a surgical blade which utilises a capacitive effect by the spacing of two conductors, the one conductor being insulated from a blade conductor, the blade conductor including a sharp edge.

A still further preferred embodiment is to utilise an electrical-to-mechanical transducer to vibrate the blade at a predetermined amplitude and frequency at any time during a surgical procedure thereby preventing build-up of debris on the surgical blade.

Reference is made to the accompanying drawings, wherein:
Figure 1 illustrates a cross-sectional view of an electro-cautery/surgical blade;
Figure 2 illustrates a sectional view taken along line 2-2 of Figure 1;
Figure 3 illustrates a sectional view taken along line 3-3 of Figure 1;
Figure 4 illustrates the electro-cautery/surgical blade wired to an electro-surgical power supply;
Figure 5 is a folded view illustrating an alternative embodiment of an electro-cautery/surgical blade;
Figure 6 illustrates a sectional view taken along line 6-6 of Figure 5;
Figure 7 illustrates a cross-sectional end view along line 7-7 of Figure 5;
Figure 8 illustrates a cross-sectional view of an alternative embodiment of a surgical blade;
Figure 9 illustrates a sectional view taken along line 9-9 of Figure 8;
Figure 10 illustrates a cross-sectional end view along line 10-10 of Figure 9;
Figure 11 illustrates another cross-sectional view of a further alternative embodiment;
Figure 12 illustrates a sectional view taken along line 12-12 of Figure 11;
Figure 13 illustrates a cross-sectional end view taken alone line 13-13 of Figure 11;
Figure 14 illustrates a plan view of a capacitive surgical blade;
Figure 15 illustrates a sectional view taken along line 15-15 of Figure 14;
Figure 16 illustrates a plan view of a capacitive surgical blade with an ultrasonic transducer;
Figure 17 illustrates a plan view of a resistive surgical blade with an ultrasonic transducer;
Figure 18 illustrates a plan view of a blade holder with an ultrasonic transducer; and
Figure 19 illustrates a view taken along line 19-19 of Figure 18.

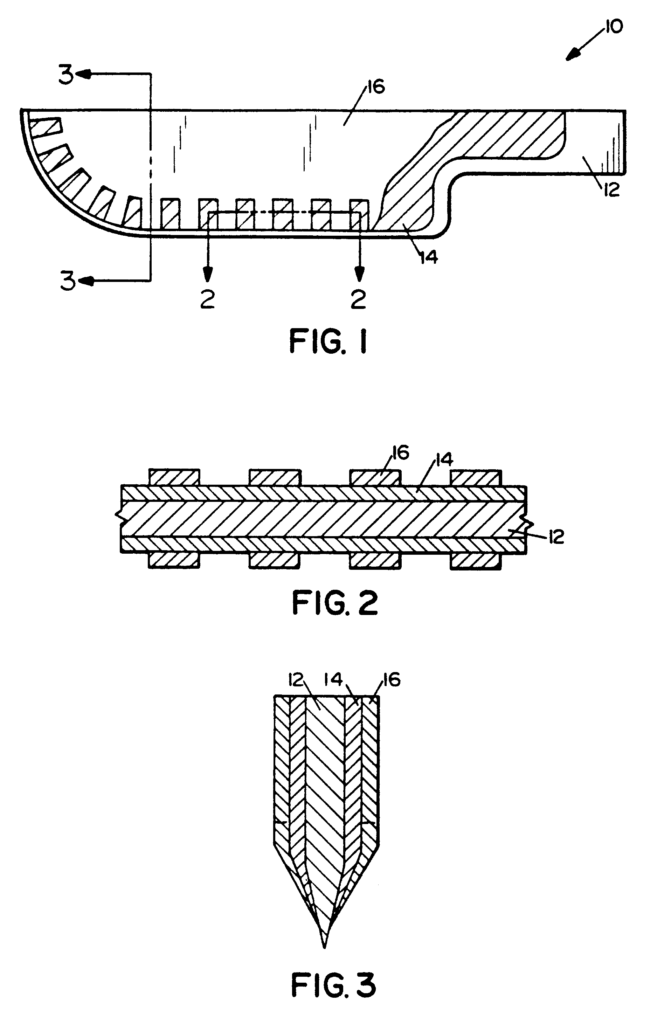

Figure 1 illustrates a cross-sectional view of the blade portion 10 of an electro-cautery/electro-surgical tool including a conductive base member 12, such as stainless steel or the like, which is capable of being ground or honed to a sharp cutting edge. Stainless steel is particualrly suitable for surgical reasons in that it maintains a sharp edge, and it can be worked in a machining and manufacturing point of view, and is a material which is recognised in a surgical and medical sense by the Food and Drug Administration. An insulation layer 14 is deposited over the conductive base 12 on both of its sides by known processes in the art. A second conductive material 16 is deposited over the insulation material 14 in a predetermined pattern. A plurality of gaps may be formed along the edge, the gaps being of a finite height and width. Processes, such as sandblasting, laser machining, chemical etching, electro-discharge machining (EDM), electron beam drilling, ion milling, grinding or the like, may be used to form a comb-like conductive electrode with opposing gaps with insulating material between the teeth. Subsequently, a sharp cutting edge is honed on the base member 12. The blade can take any predetermined geometrical configuration including one having a sharp point, a rounded point as illustrated in Figure 1, or any other geometrical figure depending upon the type of surgery as well as the surgeon's preference.

Figure 2 illustrates a view taken along line 2-2 of Figure 1 where all numerals correspond to those elements previously described. The figure illustrates the opposing comb teeth and gaps which do not necessarily have to be opposed but can be staggered.

Figure 3 illustrates an end view of Figure 1.

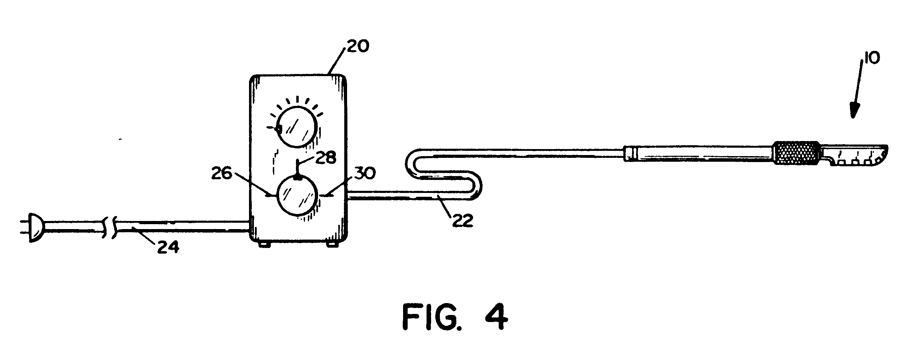

Figure 4 illustrates the electro-surgical blade 10 mounted in a holder and connected to a surgical power supply 20 by way of a surgical power cord 22 and a line cord 24. The surgical power supply is capable of operating in three modes, 26, 28 and 30. The first is a standard surgical blade mode 26 where no power is applied to the blade. The second mode is an electro-surgical mode 28 where high voltage is applied between the conductive members causing a slight discharge arc for cutting and cauterizing. The third mode involves a low voltage applied between the conductive elements where heat is produced by I²R losses solely for cauterization.

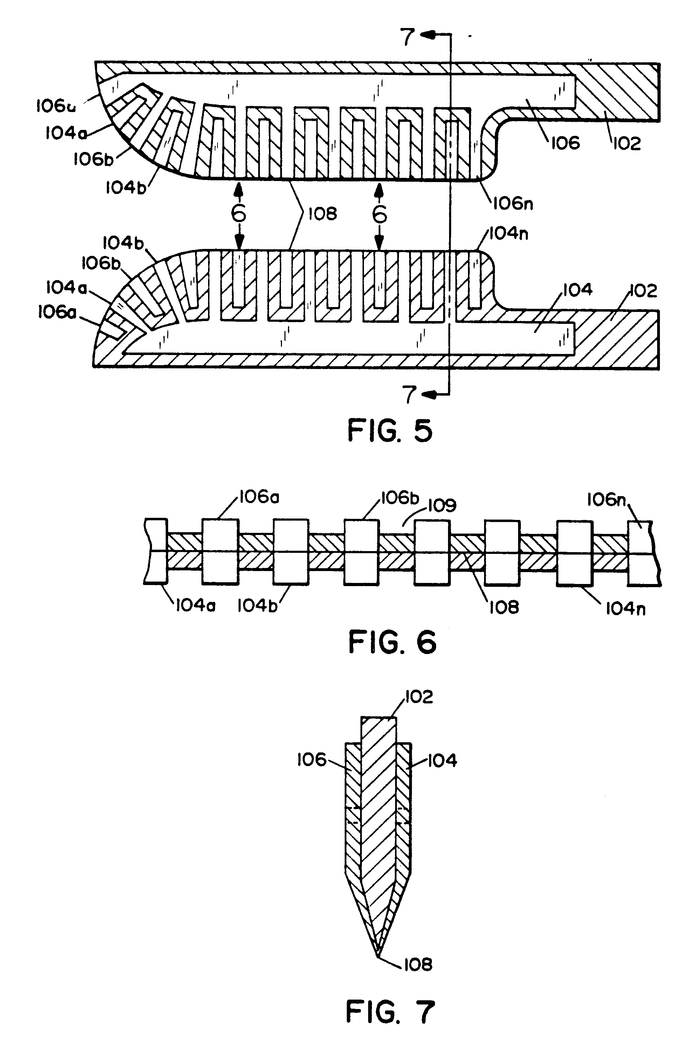

Figure 5 illustrates a folded (two-sided) view of an electrode for electro-cautery and/or electro-surgery including a non-conductive, insulative support 102, a first conductor 104 and a second conductor 106 arranged in an opposing matrix and electrically interconnected with respect to each other across the working edge 108. The conductors 104 and 106 are comb-shaped and the teeth extend to the respective opposite side of the support 102 with the extensions being interleaved with the teeth on that side. The particular tip of the cutting edge is ground to a sharp point 108.

Figure 6 illustrates a bottom view of Figure 5 showing the particular configuration of the electrodes 104a - 104n, 106a - 106n, and insulative material 102.

Figure 7 illustrates a sectional view taken along line 7-7 of Figure 5 where all numerals correspond to those elements previously described.

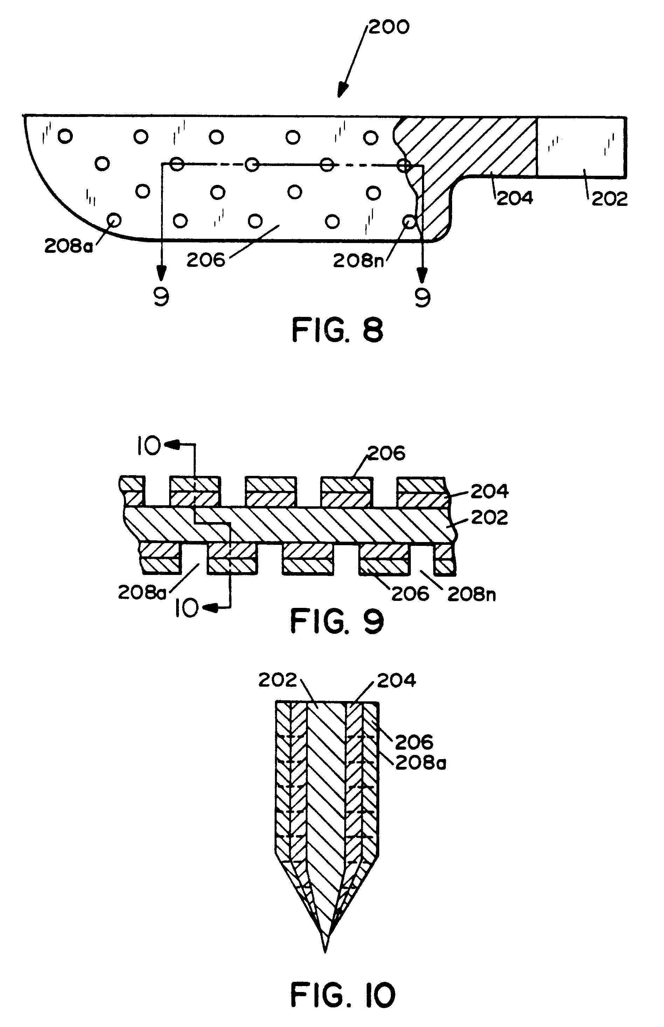

Figure 8 illustrates a cross-sectional view of an electro-cautery surgical blade 200 including a conductive base member 202, an insulation layer 204, a second conductive material 206 deposited on the layer 104, a plurality of holes 208 extending through the material 206 and the insulation 204 to the base blade 202. The holes 208a - 208n serve to distribute the electrical current in a predetermined desired fashion.

Figure 9 illustrates a sectional view taken along line 9-9 of Figure 8 where all numerals correspond to those elements previously described.

Figure 10 illustrates a cross-sectional view taken along line 10-10 in Figure 9 where all numerals correspond to those elements previously described.

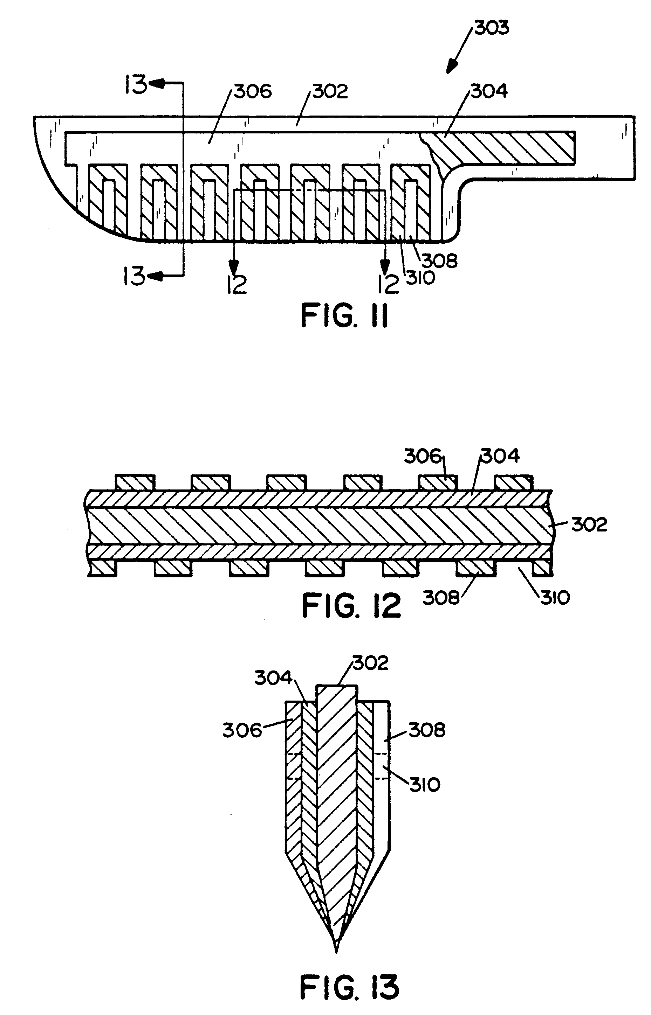

Figure 11 illustrates a cross-sectional view of an electro-surgical blade 303 including a conductive base member 302, insulation layer 304, and a second conductive material 306 deposited in layer 304 and including a plurality of wrapped-around segments 308 and gaps 310 alternating therebetween, as also illustrated in the cross-sectional view of Figure 12.

Figure 13 illustrates a view taken along line 13-13 of Figure 11 where all numerals correspond to those elements previously described.

### MODE OF OPERATION

The electro-cautery/electro-surgical blade is connected to a power supply which supplies power for three modes of operation, those modes of operation being either a mode of operation as a standard surgical blade, a mode of operation as an electro-surgical blade where a high voltage is applied between the conductive layers and the resulting discharge arc is used for cutting and cauterizing, or a mode of operation where the blade has applied to is a low voltage where heat is produced by the I²R losses for cauterization.

The insulative material can be a ceramic, glass or other non-conductive material. The conductive material can be vapor-deposited or plated and photo-etched onto the insulative non-conductive material and can be silver, gold, aluminium or the like. The base member should be high-conductivity metal which has the property of being honed to a fine sharp edge. More so, a non-conductive insulative material which may be brought to an edge, such as glass or ceramic, with a photo-etched or vapor-deposited metal thereon is particularly desirable in this disclosed group of embodiments.

### ALTERNATIVE EMBODIMENT- CAPACITIVE BLADE

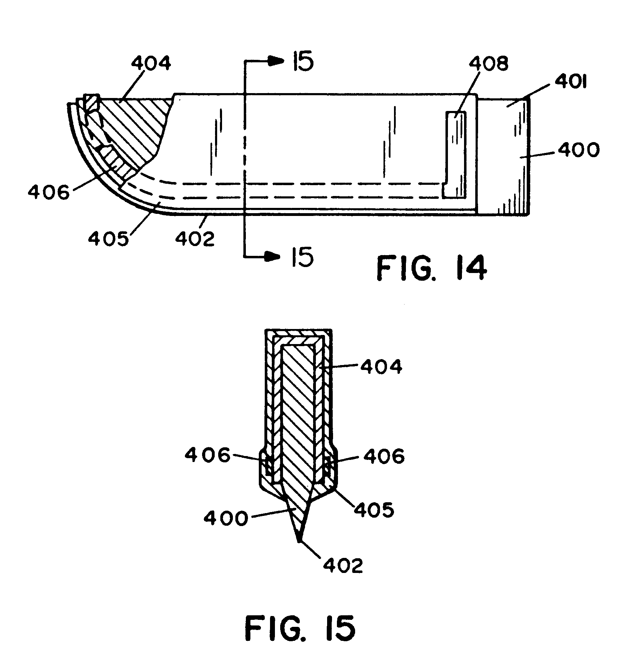

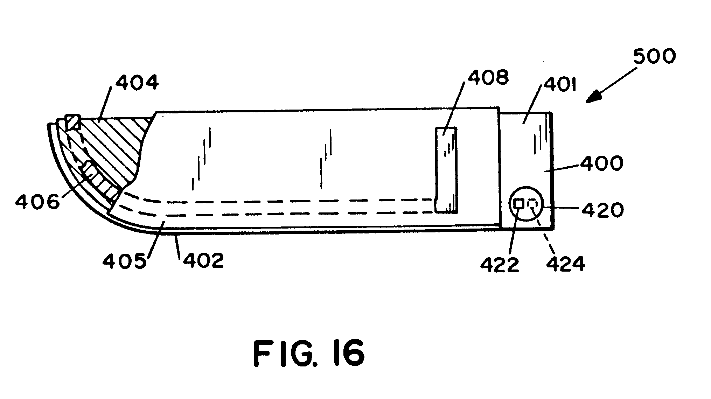

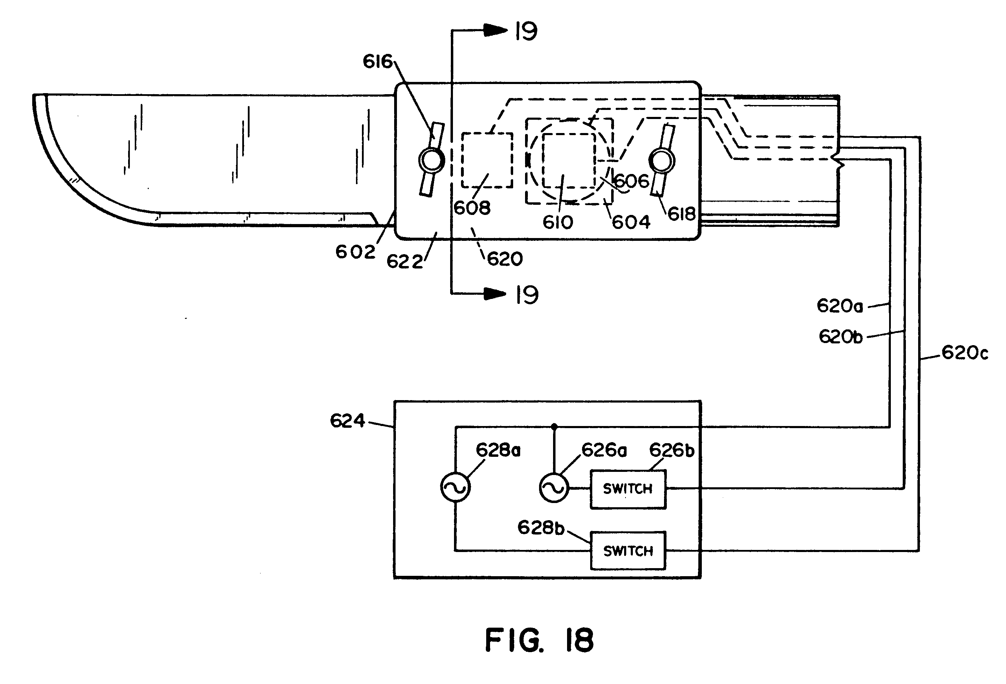

Figure 14 illustrates a side view of a capacitive electro-cautery/electro-surgical blade including a blade conductor 400 with a sharp cutting edge 402 formed on the working edge thereof. The right end of the blade when viewed in Figure 14 acts as a first electrical contact pad 401. A thick insulator 404, such as .005" to .015" (0.13 cm to 0.38 cm) thick, is positioned over the blade conductor 400, as best illustrated in the cross-sectional view of Figure 15. A narrow conductor 406 of a thickness in the range of 0.01" to 0.03" (0.02 cm to 0.08 cm) and a predetermined width is positioned slightly offset above the lowermost edge of the thick insulator 404 and, as illustrated, is a plane parallel to the blade conductor 400. The narrow conductor 406 terminates at a second contact pad 408 isolated from pad 401, and wraps around the forward upper edge of the blade as illustrated in Figure 14. A thin insulator 405 of a finite thickness of 0.001" to .002" (.002 cm to .005 cm) is positioned over the top of the narrow conductor 406 and subsequently over insulator 404, and is offset slightly below the lower edge of the thick insulator 404. The contact pads 401 and 408 provide for connection to a source of electrical current for impressing a capacitive charge across the thick blade conductor 400, insulator 404, and the thin, narrow conductor 406, thereby providing for dielectric breakdown when the blade is brought into contact with the flesh during operation. The blade can mount in a blade holder, such as that illustrated in Figure 18. The blade holder can include a screw or like for compression clamping about the blade and providing for electrical contact to the contact pads.

Figure 16 illustrates a plan view of a capacitive electro-cautery blade 400 where all numerals correspond to those elements previously described. An electrical-to-mechanical transducer, such as an ultrasonic transducer 402 is affixed to the blade and includes two contact pads 422 and 424 for supplying power from an alternating current source to the transducer 420. Contact pads 424 electrically and mechanically connects to the contact pad 401. The transducer 420 vibrates at a frequency determined by the power supply and at an amplitude which provides a cavitation effect at the selected frequency. The high frequency vibration of the blade prevents adherence of debris to the blade 400 during a surgical procedure. The transducer 420 can be powered independently or may be driven by the same power source used for energizing the blade.

### ALTERNATIVE EMBODIMENT - RESISTIVE BLADE

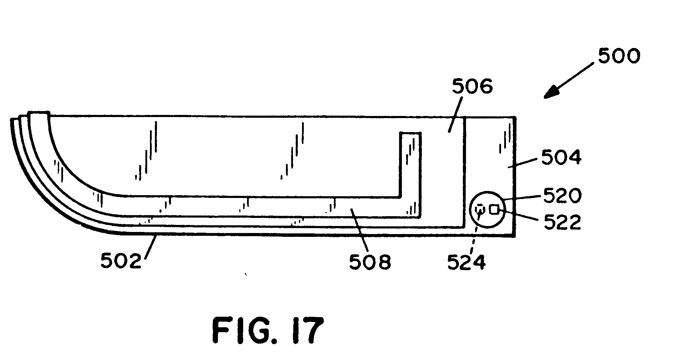

Figure 17 illustrates a plan view of a resistive electro-cautery blade 500 including a sharpened edge 502, a first contact pad area 504, a layer of insulation 506 on the conductive base but exposing the cutting edge 502, and a wrap-around exposed metal surface 508 including a second contact pad 510 deposited on or affixed to the layer 506. The resistive blade is similar to that type of blade described previously in Figures 1 to 13. A transducer element 520, including contact points 522 and 524, is positioned in intimate contact with the contact surface 504. The contact point 524 mechanically and electrically connects to the contact pad 504. The operation of the blade of Figure 17 in a vibratory mode is the same as the operation of Figures 14 to 16.

### BLADE HOLDER

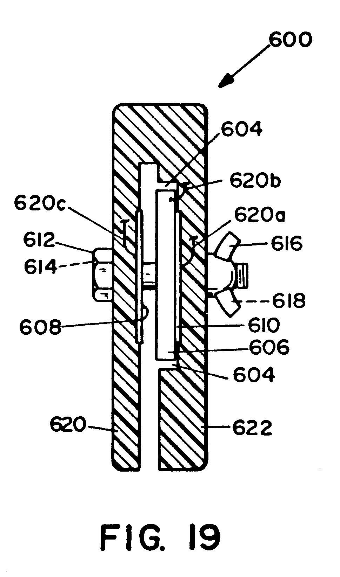

Figure 18 illustrates a plan view of a blade holder with a vibratory transducer, another alternative embodiment of the present invention. The blade holder 600 includes a slot 602 extending through a forward portion of the handle and upwardly from a lower edge of the handle into the handle as also illustrated in Figure 19. There is also provided a cavity 604 for supporting piezo-electric transducer 606 which thus locates it next to a surgical blade, all as later described in detail. The transducer 606, when mechanically compressed against the surgical blade, causes the surgical blade to vibrate in a direction transverse to the longitudinal axis of the blade during a power-on condition. In the alternative, the cavity may be configured to accept the surgical blade with the transducer permanently affixed to the surgical blade. Then, of course, appropriate electrical contact pads would have to be provided to power the ultrasonic transducer.

The blade holder 600 also includes two contact pads 608 and 610 for making contact with a surgical blade so as to be able to selectively provide power to both the blade and the transducer 606. Screws 612 and 614 and wing nuts 616 and 618 provide for compression of the two halves of the blade holder 620 and 622 so as to compress the slit 602, and hold a surgical blade in frictional engagement within the holder 600.

A power source 624 including three power cables 620a - 620c provides power to the surgical blade as well as to the transducer. The power cables include a common wire 620a, a transducer wire 620b and a surgical blade power wire 620c. The power source 624 can be switchable and pulsed as required, including a transducer waveform source 626a and switching device 626b, and blade waveform source 628a and switching device 628b.

## Claims

1. An electro-surgical instrument comprising an electro-surgical blade means (10, 200, 303, 400, 500) for electrically cutting and cauterising tissue, characterised by means (420, 520, 606) for vibrating the surgical blade means (10, 200, 303, 400, 500) at a frequency and amplitude to prevent the build-up of tissue debris thereon.

2. The surgical instrument of Claim 1 wherein said electro-surgical blade means (10, 200, 303, 400, 500) exhibits a capacitive effect.

3. The surgical instrument of Claim 1 of 2 wherein said electro-surgical blade means (10, 200, 303, 400, 500) causes heating of the tissue by a current flowing through said tissue as a resistive element.

4. The surgical instrument of Claim 1, 2 or 3 wherein said means for vibrating said surgical blade means (10, 200, 303, 400, 500) is a piezo-ceramic transducer (420, 520, 606), preferably a barium titanate piezo-ceramic transducer (420, 520, 606) optionally affixed directly to said surgical blade means (10, 200, 303, 400, 500).

5. The surgical instrument of any one of the preceding claims and further including power source means (20, 624) electrically connected to said surgical blade means (10, 200, 303, 400, 500) and to said means for vibrating said surgical blade means (10, 200, 303, 400, 500) for powering said surgical blade means (10, 200, 303, 400, 500 and said means for vibrating said surgical blade means (10, 200, 303, 400, 500).

## Patentansprüche

1. Elektrochirurgisches Instrument, umfassend eine elektrochirurgische Klingeneinheit (10, 200, 303, 400, 500) für das elektrische Schneiden und Kauterisieren von Gewebe, gekennzeichnet durch eine Einheit (420, 520, 606) zum Schwingenlassen der chirurgischen Klingeneinheit (10, 200, 303, 400, 500) auf einer Frequenz und mit einer Amplitude, bei der die Ansammlung von Geweberückständen auf der Klingeneinheit verhindert wird.

2. Chirurgisches Instrument nach Anspruch 1, wobei die elektrochirurgische Klingeneinheit (10, 200, 303, 400, 500) eine(n) kapazitive(n) Wirkung oder Effekt aufweist.

3. Chirurgisches Instrument nach Anspruch 1 oder 2, wobei die elektrochirurgische Klingeneinheit (10, 200, 303, 400, 500) eine Erwärmung des Gewebes durch einen durch das Gewebe als ohmisches oder Widerstands-Element fließenden Strom herbeiführt.

4. Chirurgisches Instrument nach Anspruch 1, 2 oder 3, wobei die Einheit zum Schwingenlassen der chirurgischen Klingeneinheit (10, 200, 303, 400, 500) ein piezokeramischer Wandler (420, 520, 606), vorzugsweise ein Batriumtitanat-Piezokeramikwandler (420, 520, 606) ist, der ggf. unmittelbar an der chirurgischen Klingeneinheit (10, 200, 303, 400, 500) befestigt ist.

5. Chirurgisches Instrument nach einem der vorangehenden Ansprüche, ferner umfassend eine elektrisch mit der chirurgischen Klingeneinheit (10, 200, 303, 400, 500) und der Einheit zum Schwingenlassen der chirurgischen Klingeneinheit (10, 200, 303, 400, 500) verbundene Stromversorgungseinheit (20, 624) zum Speisen der chirurgischen Klingeneinheit (10, 200, 303, 400, 500) und der Einheit zum Schwingenlassen der chirurgischen Klingeneinheit (10, 200, 303, 400, 500) mit Strom.

## Revendications

1. Instrument électrochirurgical comprenant un moyen servant de lame électrochirurgicale (10, 200, 303, 400, 500) pour découper et cautériser électriquement le tissu, caractérisé par un moyen (420, 520, 606) servant à faire vibrer le moyen servant de lame chirurgicale (10, 200, 303, 400, 500) à une fréquence et une amplitude déterminées, pour empêcher l'accumulation de débris de tissu sur elle.

2. Instrument chirurgical selon la revendication 1, dans lequel ledit moyen servant de lame électrochirurgicale (10, 200, 303, 400, 500) présente un effet capacitif.

3. Instrument chirurgical selon la revendication 1 ou 2, dans lequel ledit moyen servant de lame électrochirurgicale (10, 200, 303, 400, 500) provoque le chauffage du tissu au moyen d'un courant s'écoulant dans ledit tissu, servant d'élément résistif.

4. Instrument chirurgical selon la revendication 1, 2 ou 3, dans lequel ledit moyen servant à faire vibrer ledit moyen servant de lame chirurgicale (10, 200, 303, 400, 500) est un transducteur piézo-céramique (420, 520, 606), de préférence un transducteur piézo-céramique (420, 520, 606) réalisé en titanate de baryum, et pouvant être fixé directement audit moyen servant de lame chirurgicale (10, 200, 303, 400, 500).

5. Instrument chirurgical selon l'une quelconque des revendications précédentes, comprenant en outre un moyen servant de source de puissance (20, 624), relié électriquement audit moyen servant de lame chirurgicale (10, 200, 303, 400, 500) et audit moyen servant à faire vibrer ledit moyen servant de lame chirurgicale (10, 200, 303, 400, 500), pour actionner ledit moyen servant de lame chirurgicale (10, 200, 303, 400, 500) et ledit moyen servant à faire vibrer ledit moyen servant de lame chirurgicale (10, 200, 303, 400, 500).
